# EUROPÄISCHE PATENTANMELDUNG

(11) **EP 1 199 352 A2**
(43) Veröffentlichungstag der Anmeldung: **24.04.2002**
(21) Anmeldenummer: 01121803.9
(22) Anmeldetag: 11.09.2001
(51) Int. Cl.: C12M 1/107

(54) **Biofermenter**

(30) Priorität: 20.10.2000 DE 10052373
(71) Anmelder: EnviTec-Mall - Umweltsysteme GmbH & Co. KG, 48369 Saerbeck (DE)
(72) Erfinder: Ruhe, Kunibert, 49456 Bakum (Kr. Vechta) (DE); Meyer zu Rheda, Bernhard, 48329 Havixbeck (DE); Tenbrink, Jürgen, 48565 Steinfurt (DE)
(74) Vertreter: Patentanwälte Lippert, Stachow, Schmidt & Partner

(57) **Zusammenfassung**

Die Erfindung einen Biofermenter (1) mit einem wannenartig offenen Gärsubstratbehälter (2), der mit einer gasdichten Abdeckfolie (7) verschlossen ist, mit der er einen Gasspeicher (8) bildet, wobei die Abdeckfolie (7) auf der Innenseite des Gärsubstratbehälters (2) angeschlossen ist, wobei die Abdeckfolie (7) rundum an der Innenseite des Gärsubstratbehälters (2) bis unter den Flüssigkeitsspiegel (6a) des Gärsubstrats (6) reicht und an der Innenseite des Gärsubstratbehälters (2) umlaufend flüssigkeitsdicht mit diesem verbunden ist.

## Beschreibung

Die Erfindung betrifft einen Biofermenter mit einem wannenartig offenen Gärsubstratbehälter, der mit einer gasdichten Abdeckfolie verschlossen ist, mit der er einen Gasspeicher bildet, wobei die Abdeckfolie auf der Innenseite des Gärsubstratbehälters angeschlossen ist.

Der Gärsubstratbehälter eines gattungsgemäßen Biofermenters ist im Betrieb teilweise mit einem kohlenstoffhaltigen Gärsubstrat gefüllt, aus dem durch eine anaerobe Vergärung Biogas, nämlich Methan CH₄ gewonnen wird. Als Gärsubstrat kann Gülle, Klärschlamm, Maissilage, etc. dienen. Das oberhalb des Flüssigkeitsspiegels liegende Behältervolumen dient als Gasspeicher, aus dem das produzierte Gärgas entnommen werden kann.

Die Form des Gärsubstratbehälters ist beliebig. In der weiteren Beschreibung wird einfacherweise von einem zylindrischen Gärsubstratbehälter mit einem runden Grundriss ausgegangen.

Die seitliche Behälterwand des Gärsubstratbehälters besteht üblicherweise aus stahlarmiertem Beton oder Stahl. Die Abdeckfolie ist in dem Bereich des oberen Randes der Behälterwand innenseitig in dem Gärsubstratbehälter angeschlossen. Die Verbindung mit der Behälterwand ist gasdicht ausgeführt.

Bei der Fermentierung des Gärsubstrats wird unter anderem Schwefelwasserstoff H₂S und Ammoniak NH₃ frei. Kommt bei der bekannten Konstruktion aus dem Gärsubstrat aufsteigendes Ammoniak an der Behälterwand mit Wasser in Kontakt, so entsteht korrosiver Salmiakgeist NH₄OH, der die Betonoberfläche angreift. Auch die Stahlarmierung der Behälterwand kann geschädigt werden, wenn die korrosive Lösung an einer vorgeschädigten Betonoberfläche bis an die Stahlarmierung vordringen kann.

Um eine Beschädigung der Behälterwand des Gärsubstratbehälters zu verhindern, ist es bekannt, eine oder mehrere Schichten von Korrosionsschutzmittel. etc. auf der Innenseite des Gärsubstratbehälters aufzutragen. Die benötigten Korrosionsschutzmittel sind teuer. Außerdem ist es sehr arbeitsaufwendig, den Gärsubstratbehälter damit zu beschichten.

Der Erfindung liegt die Aufgabe zugrunde, einen einfach aufgebauten Biofermenter zu schaffen, der vor chemischem Angriff der Behälterwand des Gärsubstratbehälters unempfindlich ist.

Erfindungsgemäß wird die Aufgabe dadurch gelöst, dass die Abdeckfolie rundum an der Innenseite des Gärsubstratbehälters bis unter den Flüssigkeitsspiegel des Gärsubstrats reicht und an der Innenseite des Gärsubstratbehälters umlaufend flüssigkeitsdicht mit diesem verbunden ist.

Eine flüssigkeitsdichte Verbindung der Abdeckfolie mit dem Gärsubstratbehälter reicht aus, um zu verhindern, dass aufsteigende Gärgasbläschen, die sich in dem Gärsubstrat bilden, zwischen der Abdeckfolie und der Innenseite des Gärsubstratbehälters aufsteigen können. Eine gasdichte Verbindung ist hier nicht erforderlich. Die Konstruktion gewährleistet, das nirgendwo an der Betonoberfläche der Behälterwand korrosive chemische Lösungen entstehen können.

Eine vorteilhafte Weiterbildung des Biofermenters sieht vor, dass der in das Gärsubstrat reichende umlaufende Rand der Abdeckfolie zwischen einer Klemmleiste und der Innenseite des Gärsubstratbehälters festgelegt ist. Die Verbindung zwischen der Abdeckfolie und dem Gärsubstratbehälter ist einfach herstellbar und sehr sicher. Vorteilhaft ist wenigstens ein Befestigungselement vorgesehen, dass zur Verbindung der Klemmleiste mit der Innenseite des Gärsubstratbehälters dient.

Einfacherweise sind über die Länge der Klemmleiste mehrere, beispielsweise schraubenartige Befestigungselemente verteilt. Auf diese Weise ist die Abdeckfolie an ihrem umlaufenden Rand gleichmäßig gegen die Innenseite des Gärsubstratbehälters gespannt.

Vereinfacht werden kann die flüssigkeitsdichte Verbindung des freien Endes der Abdeckfolie mit der Innenseite des Gärsubstratbehälters dann, wenn gemeinsam mit der Abdeckfolie ein Dichtungselement zwischen den Gärsubstratbehälter und der Klemmleiste eingeklemmt ist. Eventuell vorhandene Zwischenräume zwischen der Abdeckfolie und dem Gärsubstratbehälter bzw. der Klemmleiste füllt das Dichtungselement aus. Diese Maßnahme erleichtert insbesondere die Abdichtung gegen eine raue oder unebene Betonoberfläche der Behälterwand des Gärsubstratbehälters. Bei Verwendung eines Dichtungselementes kann die Betonwand des Gärsubstratbehälters, was deren Ebenheit angeht, mit relativ geringer Qualität und somit kostengünstig hergestellt werden. Das Dichtungselement gleicht die Rauhigkeit und Unebenheit der Innenseite beziehungsweise Innenwand des Gärgasbehälters aus und schafft eine flüssigkeitsdichte Verbindung zwischen der Abdeckfolie und dem Gärsubstratbehälter. Selbstverständlich kann das Dichtungselement während der Montage in Form einer Dichtungsmasse aufgetragen werden, die im montierten Zustand des Biofermenters aushärtet.

Bei einem Biofermenter mit wenigstens einer gasdicht verschließbaren Durchführung zur Beschickung und/oder zur Entleerung von Gärsubstrat ist es günstig, wenn die Durchführung auf der Innenseite des Gärsubstratbehälters eine Anschlusseinrichtung aufweist, mit der die Abdeckfolie gasdicht mit der Durchführung verbindbar ist. Das gleiche gilt auch für eine als Mannloch dienende Durchführung. Bei einem Biofermenter nach dem Stand der Technik ist zwischen der Behälterwand und der Durchführung eine Fuge vorhanden, die mit Dichtungsmittel abgedichtet werden muss. Durch die einfache Maßnahme, eine Anschlusseinrichtung vorzusehen, erübrigt sich die Abdichtung der Fuge. Als weiterer Vorteil dieser Maßnahme wird es angesehen, dass die Öffnung des Gärsubstratbehälters mit reichlich Spiel für das Einfügen der Durchführung versehen sein kann. Die Öffnung kann daher kostengünstig mit grober Fertigungstoleranz hergestellt sein.

Einfacherweise weist die Anschlusseinrichtung der Durchführung einen die Durchführung radial umgebenden Klemmflansch auf, in dem die Abdeckfolie gasdicht einspannbar ist. Der Klemmflansch kann beispielsweise zwei Flanschteile aufweisen, von denen das eine radial umlaufend gasdicht mit der Durchführung verbunden ist und sich das andere lose über ein freies Ende der Durchführung schieben lässt. Im montierten Zustand ist die Abdeckfolie zwischen die beiden Flanschteile des Klemmflansches gelegt und sind diese mit wenigstens einem Befestigungselement gegeneinander verspannt, so dass die Abdeckfolie gasdicht eingeklemmt ist.

Die Handhabung des Biofermenters, insbesondere bei der Inbetriebnahme lässt sich verbessern, wenn die Abdeckfolie des Gärsubstratbehälters mit wenigstens einem Spannelement über dem Gärsubstratbehälter aufgespannt ist. Dies verhindert es, dass die Abdeckfolie zu dem Gärsubstrat hin durchhängen kann.

Zweckmäßig sind mehrere Spannelemente einerseits an der dem Gasspeicher abgewandten Außenseite der Abdeckfolie angebracht und anderen Endes mit dem Gärsubstratbehälter verbunden. Bei einer Inbetriebnahme lässt sich die Abdeckfolie sehr einfach mit den Spannelementen an dem Gärsubstratbehälter aufhängen.

Im Betrieb wird die Abdeckfolie durch einen sich in dem Gasspeicher aufbauenden Überdruck wie ein gasgefüllter Ballon nach außen gewölbt. Bei einer Entnahme von Gärgas stellen die Spannelemente sicher, dass die Abdeckfolie nicht auf das Gärsubstrat oder den Boden des Gärsubstratbehälters herunterfällt. Es ist wichtig, dies zu verhindern, da sich der Biofermenter mit einer bis an den Boden oder an das Gärsubstrat heranreichenden Abdeckfolie nur sehr schlecht beschicken und in Betrieb nehmen lassen würde.

Einen weiteren Nutzen bringt es, wenn ein Stützelement vorgesehen ist, dass die Abdeckfolie auf der dem Gärsubstrat zugewandten Seite unterstützt. Letzteres Stützelement ist somit innerhalb des Gasspeichers vorgesehen. Während die Spannelemente die Abdeckfolie am äußeren Umfang aufspannen, verhindert das Stützelement an einer innerhalb des Gasspeichers liegenden Stelle ein Herunterhängen der Abdeckfolie.

Dann, wenn das maximale Volumen des Gasspeichers veränderbar sein soll, ist das Stützelement günstigerweise in vertikaler Richtung längenveränderlich. Dies vereinfacht es, den Biofermenter anzupassen an Gärsubstrate mit unterschiedlich starker Gärgasbildung. Außerdem kann das Gasspeichervolumen bei gegebener Gärgasbildung entsprechend der erwarteten Entnahmemengen individuell angepasst werden.

Bei einer Ausführungsform eines Biofermenters mit einem wannenartig offenen Gärsubstratbehälter, der mit einer gasdichten Abdeckfolie verschlossen ist, mit der er einen Gasspeicher bildet, wobei die Abdeckfolie auf der Innenseite des Gärsubstratbehälters angeschlossen ist, kann vorteilhaft ein Ballast die Abdeckfolie beaufschlagen, wodurch ein konstanter Druck in dem Gasspeicher erzeugbar ist.

Nachstehend ist die Erfindung in einer Zeichnung beispielhaft veranschaulicht und anhand der einzelnen Figuren detailliert beschrieben. Es zeigen:
- Fig. 1: einen Ausschnitt eines Biofermenters in Schnittdarstellung,
- Fig. 2: einen Ausschnitt eines alternativen Biofermenters in Schnittdarstellung.

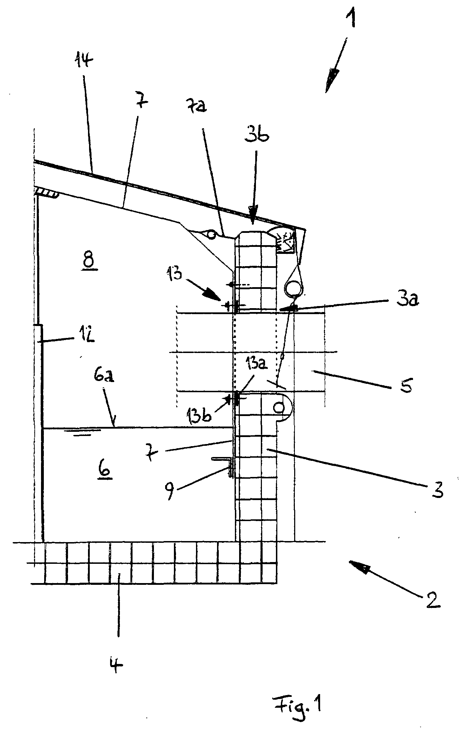

Nach der Zeichnung weist der Biofermenter 1 einen Gärsubstratbehälter 2 mit einer zylindrischen Behälterwand 3 aus stahlarmiertem Beton auf. Vereinfachend zeigt Fig. 1 der Zeichnung eine Schnittdarstellung eines Teils des Biofermenters. Ebenfalls dargestellt ist der Behälterboden 4 des Gärsubstratbehälters 2, der im vorliegenden Ausführungsbeispiel auch aus Beton besteht.

Gemäß der Zeichnung ist der Gärsubstratbehälter 2 mit einem Gärsubstrats 6 beschickt. Der Flüssigkeitsspiegel 6a des Gärsubstrats 6 steht auf einem Niveau zwischen der Durchführung 5 und dem Behälterboden 4 des Gärsubstratbehälter 2.

Der Gärsubstratbehälter 2 ist mit einer gasdichten Abdeckfolie 7 verschlossen, die ihrerseits gasdicht mit der Innenseite der Behälterwand 3 des Gärsubstratbehälters 2 verbunden ist. Auf diese Weise bildet der Gärsubstratbehälter 2 gemeinsam mit der Abdeckfolie 7 einen Gasspeicher 8. Im Betrieb wird Gärgas in einer solchen Menge gebildet, dass durch den Druck in dem Gasspeicher 8 die Abdeckfolie 7 ballonartig gewölbt wird.

An dem freien umlaufenden Rand 3b der Behälterwand 3 sind umfänglich mehrere Spannelemente in Form von Spannbändern 7a vorgesehen, die die Abdeckfolie 7 über dem Gärsubstratbehälter 2 aufspannen und über der Öffnung des wannenartigen Gärsubstratbehälters 2 halten.

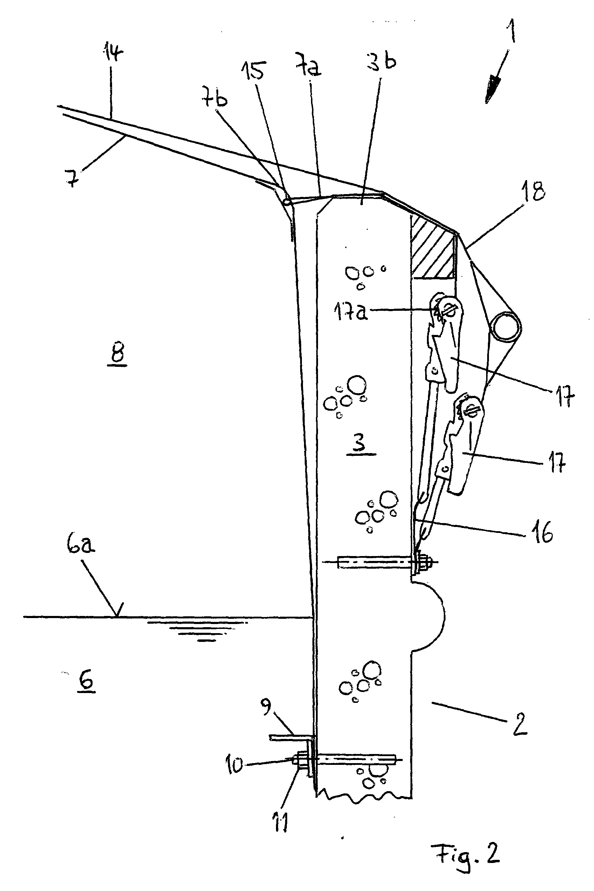

Die Innenseite der Behälterwand 3 des Gärsubstratbehälters 2 ist größtenteils mit der Abdeckfolie 7 verkleidet. Nicht verkleidet ist nur ein Teil der Behälterwand 3, der sich unterhalb des Flüssigkeitsspiegels 6a des Gärsubstrats 6 befindet. Die Abdeckfolie 7 schmiegt sich gemäß Fig. 1 an die Innenseite der Behälterwand 3 des Gärsubstratbehälters 2 an. In der alternativen Ausführungsform gemäß Fig. 2 hingegen ist die Abdeckfolie in einem kurzen Abstand vor der Innenseite der Behälterwand 3 des Gärsubstratbehälters 2 abgespannt.

Da das dem Behälterboden 4 zugewandte umlaufende Ende der Abdeckfolie 7 im befüllten Zustand des Gärsubstratbehälters 2 bis unter den Flüssigkeitsspiegels 6a des Gärsubstrats 6 reicht, bildet die Abdeckfolie 7 eine Art Glocke, unter der das Gärgas eingeschlossen ist. Der nicht mit Gärsubstrat 6 benetzte Teil des Gärsubstratbehälters 2 ist hermetisch von dem entstehenden Gärgas getrennt.

Die Behälterwand 3 ist mit einer Öffnung 3a versehen, in der eine rohrförmige Durchführung 5 zum Beschicken und Entleeren des Gärsubstratbehälters 2 angeordnet ist. Zum einfachen Beschicken und Entleeren kann ein Biofermenter eine Vielzahl von Durchführungen 5 aufweisen, die in unterschiedlichem Abstand vom Behälterboden 4 angeordnet sind. Die Durchführung 5 ist mit einer Verschlusseinrichtung 5a gasdicht verschließbar, damit auch dort kein Gärgas entweichen kann.

Das freie umlaufende Ende der Abdeckfolie 7 ist mit einer Klemmleiste 9 von L-förmigem Querschnitt gegen die zylindrische Behälterwand 3 gespannt, um die Flüssigkeitsdichtigkeit sicherzustellen. Dies verhindert außerdem, dass der Zwischenraum zwischen der Abdeckfolie 7 und der Behälterwand 3 mit Gärsubstrat 6 verschmutzt wird, das an dieser Stelle bei einer Reinigung des Gärsubstratbehälters 2 nur schlecht zu entfernen wäre. Um die Klemmleiste einfach anbringen zu können, weist die zylindrische Behälterwand 3 Befestigungselemente mit Gewindebolzen 10 auf, die von der Behälterwand 3 hervorstehen. Die Klemmleiste 9 weist Durchgangsbohrungen auf, die auf die Gewindebolzen 10 der Behälterwand 3 aufgesteckt sind. Eine Spannmutter 11 spannt die Abdeckfolie 7 gegen die Behälterwand 3.

Etwa in dem Zentrum des Behälterbodens 4 des Gärsubstratbehälters 2 ist ein Stützelement 12 mit einem längenveränderbaren Auflager für die Abdeckfolie angeordnet. Das Stützelement 12 wirkt mit den an der zylindrischen Behälterwand 3 angeschlossenen Spannbändern 7a zusammen und spannt die Abdeckfolie 7 wie ein Zelt auf. Üblicherweise weist der Gasspeicher ein festes Speichervolumen auf. Es besteht alternativ jedoch auch die Möglichkeit, durch eine Längenveränderung des Stützelements 12 beziehungsweise des Auflagers gegenüber der in Fig. 1 gezeichneten Länge die Abdeckfolie 7 zum Behälterboden 4 des Gärsubstratbehälters 2 hin abzusenken oder anzuheben und das Volumen des Gasspeichers 8 zu verändern. Die Abdeckfolie 7 wird stets mit Hilfe der Spannbänder 7a nachgespannt und gestrafft.

Die Durchführung 5 weist zur einfachen Verbindung mit der Abdeckfolie 7 einen Klemmflansch 13 auf. Der Klemmflansch 13 ist aus zwei Flanschteilen 13a und 13b gebildet, von denen ein Flanschteil 13a an dem Umfang der rohrförmigen Durchführung 5 gasdicht angeschlossen ist. Das andere Flanschteil 13b ist lose über das in den Gasspeicher 8 ragende Ende der Durchführung 5 schiebbar. In die Abdeckfolie 7 ist ein Loch geschnitten, das etwa den Querschnitt der Durchführung 5 aufweist. Der Rand des Lochs der Abdeckfolie 7 wird während der Montage zwischen die Flanschteile 13a und 13b des Klemmflansches 13 gelegt. Im montierten Zustand spannen Verbindungselemente, beispielsweise Schrauben, die Flanschteile 13a und 13b gegeneinander und verbinden die Abdeckfolie 7 gasdicht mit der Durchführung 5.

Der lichte Querschnitt der Durchführung 5 ist über eine Verschlusseinrichtung in Form einer Klappe 5a gasdicht verschließbar.

Um die Abdeckfolie 7 vor Witterung zu schützen, ist ein zusätzliches Dach 14 vorgesehen. In dem dargestellten Ausführungsbeispiel ist das Dach 14 nach Art einer Zeltplane ausgebildet. Es ragt in radialer Richtung über das freie Ende der zylindrischen Behälterwand 3 hinaus und ist an der Außenseite des Gärsubstratbehälters 2 festgespannt.

Gemäß Fig. 2 ist eine Ausführungsform des Biofermenters dargestellt, dessen Abdeckfolie 7 einen umlaufenden Haltering 15 aufweist. An der Außenseite der Abdeckfolie 7 sind Haltelaschen 7b angebracht, in denen der Haltering 15, wie ein Gürtel in den Gürtelschlaufen einer Hose, gehalten ist. Die Spannbänder 7a halten die Abdeckfolie 7 an dem Haltering 15. Sie sind über das freie Ende der Behälterwand 3 zur Außenseite des Gärsubstratbehälters 2 geführt und dort an einer Befestigungsplatte 16 verankert.

Zur Einstellung der Spannkraft der Spannbänder 7a sind Spannratschen 17 vorgesehen. Mit den Spannratschen 17 kann mehr oder weniger Spannband 7a auf einer Spannbandtrommel 17a aufgewickelt werden, so dass sich stets die gewünschte Spannkraft ergibt. Jede Spannratsche 17 weist eine Rücklaufsicherung auf. Nach Fig. 2 weist eine Rücklaufsicherung Sperrklinken auf, die die Drehrichtung der Spannbandtrommel 17a in Rücklaufrichtung sperren. Für eine Wegnahme der Spannkraft muss die Sperrklinke entsperrt werden.

Für das zeltplanenartige Dach 14 des Biofermenters ist ebenfalls ein an der Außenseite des Gärsubstratbehälters 2 verankertes Spannband 18 vorgesehen. Letzteres weist eine Spannratsche 17 der gleichen Art auf, wie die Spannbänder 7a der Abdeckfolie 7.

### Bezugzeichenliste

- 1: Biofermenter
- 2: Gärsubstratbehälter
- 3: Behälterwand
- 3a: Öffnung
- 3b: umlaufender Rand
- 4: Behälterboden
- 5: Durchführung
- 6: Flüssigkeitsspiegel
- 7: Abdeckfolie
- 7a: Spannband
- 7b: Haltelasche
- 8: Gasspeicher
- 9: Klemmleiste
- 10: Gewindebolzen
- 11: Spannmutter
- 12: Stützelement
- 13: Klemmflansch
- 13a: Flanschteil
- 13b: Flanschteil
- 14: Dach
- 15: Haltering
- 16: Befestigungsplatte
- 17: Spannratsche
- 17a: Spannbandtrommel
- 18: Spannband

## Patentansprüche

1. Biofermenter (1) mit einem wannenartig offenen Gärsubstratbehälter (2), der mit einer gasdichten Abdeckfolie (7) verschlossen ist, mit der er einen Gasspeicher (8) bildet, wobei die Abdeckfolie (7) auf der Innenseite des Gärsubstratbehälters (2) angeschlossen ist, **dadurch**
**gekennzeichnet, dass** die Abdeckfolie (7) rundum an der Innenseite des Gärsubstratbehälters (2) bis unter den Flüssigkeitsspiegel (6a) des Gärsubstrats (6) reicht und an der Innenseite des Gärsubstratbehälters (2) umlaufend flüssigkeitsdicht mit diesem verbunden ist.

2. Biofermenter nach Anspruch 1, **dadurch gekennzeichnet, dass** der in das Gärsubstrat (6) reichende umlaufende Rand der Abdeckfolie (7) zwischen einer Klemmleiste (9) und der Innenseite des Gärsubstratbehälters (2) festgelegt ist.

3. Biofermenter nach Anspruch 2, **dadurch gekennzeichnet, dass** wenigstens ein Befestigungselement vorgesehen ist, das zur Verbindung der Klemmleiste (9) mit der Innenseite des Gärsubstratbehälters (2) dient.

4. Biofermenter nach Anspruch 2 oder 3, **dadurch gekennzeichnet, dass** die Abdeckfolie (7) gemeinsam mit einem Dichtungselement zwischen dem Gärsubstratbehälter (2) und der Klemmleiste (9) eingeklemmt ist.

5. Biofermenter nach einem der Ansprüche 1 bis 4 mit wenigstens einer gasdicht verschließbaren Durchführung (5) zur Beschickung und/oder zur Entleerung von Gärsubstrat (6), **dadurch gekennzeichnet, dass** die Durchführung (5) auf der Innenseite des Gärsubstratbehälters (2) eine Anschlusseinrichtung aufweist, mit der die Abdeckfolie (7) gasdicht mit der Durchführung (5) verbindbar ist.

6. Biofermenter nach Anspruch 5, **dadurch gekennzeichnet, dass** die Anschlusseinrichtung (7) einen die Durchführung (5) radial umgebenden Klemmflansch (13) aufweist, in dem die Abdeckfolie (7) gasdicht einspannbar ist.

7. Biofermenter nach einem der Ansprüche 1 bis 6, **dadurch gekennzeichnet, dass** die Abdeckfolie (7) des Gärsubstratbehälters (2) mit wenigstens einem Spannelement über dem Gärsubstratbehälter (2) aufgespannt und so verhindert ist, dass die Abdeckfolie (7) zu dem Gärsubstrat (6) hin durchhängt.

8. Biofermenter nach einem der Ansprüche 1 bis 7, **dadurch gekennzeichnet, dass** ein Stützelement (12) vorgesehen ist, das die Abdeckfolie (7) auf der dem Gärsubstrat (6) zugewandten Folienseite unterstützt.

9. Biofermenter nach Anspruch 8, **dadurch gekennzeichnet, dass** das Stützelement (12) in vertikaler Richtung längenveränderlich und das maximale Volumen des Gasspeichers (8) dadurch veränderbar ist.

10. Biofermenter (1) nach Oberbegriff des Anspruchs 1, **dadurch gekennzeichnet, dass** ein Ballast die Abdeckfolie (7) beaufschlagt und dadurch ein konstanter Druck in dem Gasspeicher (8) erzeugbar ist.
